# EUROPEAN PATENT SPECIFICATION

(11) **EP 2 388 339 B1**
(45) Date of publication and mention of the grant of the patent: **17.02.2016**
(21) Application number: 11164434.0
(22) Date of filing: 02.05.2011
(51) Int. Cl.: C12Q 1/68

(54) **Method for determination of and medicament for influencing the activity of the immune system**
Verfahren zur Bestimmung von und Medikament zur Beeinflussung der Aktivität des Immunsystems
Procédé de détermination et médicament pour influencer l'activité d'un système immunitaire

(30) Priority: 30.04.2010 EP 10161687
(43) Date of publication of application: 23.11.2011
(73) Proprietor: Medizinische Hochschule Hannover, 30625 Hannover (DE)
(72) Inventor: Blasczyk, Rainer, 30916 Isernhagen (DE); Ferreira de Figueiredo, Constanca, 31535 Neustadt am Rübenberge (DE); Gras, Christine, 30171 Hannover (DE)
(74) Representative: Taruttis, Stefan Georg

(56) References cited:
- WO-A1-2006/013614
- WO-A2-2005/104785
- US-A1- 2005 042 636
- REDDY PAVAN: "Pathophysiology of acute graft-versus-host disease.", HEMATOLOGICAL ONCOLOGY DEC 2003 LNKD- PUBMED:14735553, vol. 21, no. 4, December 2003 (2003-12), pages 149-161, XP002589009, ISSN: 0278-0232
- LI X C ET AL: "IL-15 and IL-2: a matter of life and death for T cells in vivo.", NATURE MEDICINE JAN 2001 LNKD- PUBMED:11135625, vol. 7, no. 1, January 2001 (2001-01), pages 114-118, XP002589010, ISSN: 1078-8956
- CHRISTIANE GRAS ET AL.: "Secreted Semaphorin 5A Activates Immune Effector Cells and Is a Biomarker for Rheumatoid Arthritis", ARTHRITIS & RHEUMATOLOGY, vol. 66, no. 6, 38425 , pages 1461-1471, DOI: 10.1002

### SEQUENCE LISTING

<110> Medizinische Hochschule Hannover
<120> Method for the determination of and medicament for influencing the activity of the immune system
<130> M1029-N-EP
<160> 11
<170> PatentIn version 3.5
<210> 1
   <211> 17
   <212> DNA
   <213> Artificial Sequence
<220>
   <223> /note="SEQ ID NO 1: synthetic primer"
<400> 1
   caacggcggc cactatg 17
<210> 2
   <211> 15
   <212> DNA
   <213> Artificial Sequence
<220>
   <223> /note="SEQ ID NO 2: synthetic primer"
<400> 2
   ccaggcgggc tttgc 15
<210> 3
   <211> 18
   <212> DNA
   <213> Artificial sequence
<220>
   <223> /note="SEQ ID NO 3: synthetic probe"
<400> 3
   caacgattcc gatacaca 18
<210> 4
   <211> 20
   <212> DNA
   <213> Artificial Sequence
<220>
   <223> /note="SEQ ID NO 4: synthetic primer"
<400> 4
   ttcagcaatg gagctttcca 20
<210> 5
   <211> 18
   <212> DNA
   <213> Artificial Sequence
<220>
   <223> /note="SEQ ID NO 5: synthetic primer"
<400> 5
   caaccccagc cgttgaac 18
<210> 6
   <211> 19
   <212> DNA
   <213> Artificial Sequence
<220>
   <223> /note="SEQ ID NO 6: synthetic probe"
<400> 6
   aagcagcaac aactatata 19
<210> 7
   <211> 18
   <212> DNA
   <213> Artificial Sequence
<220>
   <223> /note="SEQ ID NO 7: synthetic primer"
<400> 7
   atgatgatat cgccgcgc 18
<210> 8
   <211> 16
   <212> DNA
   <213> Artificial Sequence
<220>
   <223> /note="SEQ ID NO 8: synthetic primer"
<400> 8
   gccttgcaca tgccgg 16
<210> 9
   <211> 15
   <212> DNA
   <213> Artificial Sequence
<220>
   <223> /note="SEQ ID NO 9: synthetic probe"
<400> 9
   cgtcgtcgac aacgg 15
<210> 10
   <211> 11825
   <212> DNA
   <213> Human semaphorin 5A
<400> 10
   gctctccgcc ccgcgctctc cgcgcccgct cgccccgcgc cgatctactt gccgggaagg      60
   cggcgccggt ggcggctgct ctccctgagc ccgctcccga gcgctgcttt cccgccgcgg     120
   gtgggcttcg cagcctcagg ccagccgcgg cccttggccc gctgcagccc cggccctcca     180
   ccttccccgt gcaggggcgg cccggccagt gtcgctcatc ccgggacgct cccttctccc     240
   acccaggact gccccgcgga gctggcttgg acacccaact ttgccacctc gagggtcgtc     300
   tctgctgggc gcgaacctgc ccacccaccg gttggccgcg cgctcgggga ccgtgctcgt     360
   ggcccccaag ccggtgcccc cattctggaa ctcagcgagt agggggcggc tctggggaag 420
   tggcaggggg cggctgcagc tgctgcctcc acttccctag ccaggtgctg aagaggatcc 480
   tcggagccgc tctggccccc aggcgctgga tgactggcac cagcgctcct cgcacctgtg 540
   ttggtgtgtg agacttgggc tggagtgccc acgtggctgt ggagtcagtg tgattcatga 600
   ttgaggaaac gcgtcctcca tcctctctct ccttggcact ttccacacat gaggagaaga 660
   agagcttctg tttagaagac acgtgcccag agtcagaggc cccttgccca ccatgaaggg 720
   aacctgtgtt atagcatggc tgttctcaag cctggggctg tggagactcg cccacccaga 780
   ggcccagggt acgactcagt gccagagaac cgagcatcca gtcatctcct ataaagaaat 840
   tggcccctgg ttacgggagt tcagagcgaa gaatgctgtg gatttctcgc agttaacatt 900
   tgacccagga cagaaagaac ttgttgtagg agcaagaaac tacctcttca ggttacagct 960
   tgaggatctg tctcttatcc aggctgtgga atgggagtgt gatgaagcta ccaaaaaggc 1020
   ctgttacagc aaaggcaaat caaaggagga atgtcagaac tacatccggg tgcttctggt 1080
   gggtggcgac cggttattca cctgtgggac caatgcattc acgcctgtct gcaccaaccg 1140
   ctcgttgagc aacctgactg agatccatga tcagatcagt ggcatggccc gctgtcccta 1200
   cagtccccag cacaattcca cagcgctcct cacagctggt ggggagctct atgctgctac 1260
   agccatggat tttccaggac gtgatcctgc catttaccga agcctaggca ttttacctcc 1320
   tctccgcacg gcgcagtaca actccaaatg gctcaatgag ccaaactttg tgtcatctta 1380
   tgacatcgga aattttacct acttcttttt ccgagaaaat gcagtagagc atgactgtgg 1440
   gaaaacagtg ttctccagag ctgcccgggt gtgcaagaac gatattggtg ggcgcttcct 1500
   gctggaagac acctggacca cattcatgaa ggctcgcctg aactgctccc gtcctgggga 1560
   agtccccttt tactacaacg aattgcagag tactttcttc ctgcctgagc tggatttgat 1620
   ctatggcatc tttaccacca atgtgaacag cattgcggcc tcagctgtgt gcgtcttcaa 1680
   cctgagcgcc atcgcgcagg ccttctctgg gcccttcaag taccaagaaa actcgcgctc 1740
   ggcctggcta ccgtatccca acccaaaccc ccacttccag tgtggcaccg tggaccaggg 1800
   cctgtacgtg aacctgaccg agagaaatct gcaggatgct cagaagttca ttctgatgca 1860
   tgaggtggta cagccagtga ccacagtgcc ctccttcatg gaggacaata gccgcttttc 1920
   ccacgtggca gtcgacgtgg tgcagggcag agaagcgctc gtccacatca tctatttggc 1980
   cacagattac ggaaccatta agaaagtgcg ggtacccctg aatcagacct caagcagctg 2040
   tttgctggaa gagattgagc tcttccctga gaggcggagg gagcccatca ggagcctgca 2100
   gatcctgcac agccagagtg tcctgttcgt gggcctgcgg gagcacgtgg tcaagatccc 2160
   cctgaagagg tgccagttct accgcacacg cagcacctgc attggggccc aggaccctta 2220
   ctgtggctgg gatgtggtaa tgaagaaatg cacaagcctg gaggagagcc tgagcatgac 2280
   gcagtgggaa cagagcatct ctgcgtgtcc gaccaggaat ctcaccgtgg atgggcactt 2340
   tggtgtgtgg tctccgtgga cgccttgcac gcacacagat ggcagcgccg tgggatcctg 2400
   cctctgtcga acccgctcct gcgacagccc ggccccgcag tgtggtggct ggcagtgcga 2460
   gggccctggc atggagatcg ccaactgttc caggaacgga ggctggactc cctggacctc 2520
   gtggtctccc tgcagcacta cctgtgggat cggcttccag gtgcggcagc gctcctgcag 2580
   caaccccact cccaggcacg ggggccgggt gtgcgtggga cagaaccgcg aggaaagata 2640
   ctgcaatgaa catttgctat gtcccccaca catgttctgg acaggctggg gtccttggga 2700
   acggtgcaca gcccaatgcg ggggtggcat tcaagctcgc cgcaggatct gtgagaatgg 2760
   gcctgactgt gcaggctgca atgtggagta ccagtcttgc aacaccaacc cgtgtcctga 2820
   gctgaagaag accacgccct ggacaccctg gacacctgtc aacatctctg acaacggcgg 2880
   ccactatgag caacgattcc gatacacatg caaagcccgc ctggctgatc cgaatttgct 2940
   ggaagtggga agacagagaa tcgaaatgcg gtactgttct agcgacggca ccagtggctg 3000
   ctccacagat gggctttctg gggatttcct gcgtgctggg agatactctg cccacacggt 3060
   caacggggct tggtcagcct ggacgtcgtg gtcacagtgc agccgtgact gcagcagggg 3120
   cattcggaac cggaagcgtg tttgcaacaa ccccgaaccc aagtatgggg gaatgccttg 3180
   ccttggccca tctctggaat accaggaatg caacattttg ccctgcccag tggatggcgt 3240
   gtggtcttgc tggtccccct ggacaaaatg ttcagcaaca tgcggcggtg gacactatat 3300
   gaggacccgc tcttgctcca atccagcccc ggcctatgga ggggacatct gcctggggct 3360
   gcacacagaa gaggcactct gcaacacgca gccctgccca gagagctggt cggagtggtc 3420
   ggactggtct gagtgtgaag cctctggcgt ccaagtccgc gcccgccagt gcatcctcct 3480
   gttccccatg ggcagccagt gctccgggaa caccacggag agccggccgt gtgtgtttga 3540
   ctctaatttc atcccagaag tatctgtggc aagatccagt agcgtagaag agaaaaggtg 3600
   tggagagttc aacatgttcc acatgatcgc cgtggggctg agcagctcca tcctcggctg 3660
   cctcctcacc ctgctcgtct atacttactg ccagcggtac cagcagcaat cccacgatgc 3720
   gactgtcatc caccccgtct cacctgcccc ccttaatacc agcataacca accacatcaa 3780
   caaactggac aagtacgact cggtggaggc catcaaggca tttaacaaaa acaacttgat 3840
   cctagaggaa agaaacaaat acttcaaccc acatctcact gggaagacct attctaatgc 3900
   ctactttaca gatctcaata attatgatga gtactaacag ctttcatgtt tttggcttct 3960
   tgtaaatccc cagttcctca aggcctgtgc cccatgactg cccatgtttc tgaggcttca 4020
   gagtcgaagt ttggatacat ttcaagtgca tttcaagcca ccagagtgtc ccattgctgc 4080
   caaaaataca cgtctttaaa agcaacaaaa attgaaataa gacatcgtga aaatcttgac 4140
   cattgttgaa tgagccaggg tgtgaagttt ttaattgtgt tcatcctatt tttctgacaa 4200
   gtccattggt ttgttttttg agcattattt tataaatgtg ccacccacat tggaaggagt 4260
   ctttctttag aactttggag tgtaaatctt catgatgttg taattcaaga aaataggcac 4320
   tttctctgaa agacctgctc cttccacaag aagtgcatag gtccataata tttcataaaa 4380
   tgaagaaaaa gaatgtggcc aaacaattat tcaccatgga ttgcccaact ttccaaatct 4440
   ggataaagct gtgggattct tggaagcagc ttgagtgttt tcatcttgcc tgggaagccc 4500
   aggaattcca cctggtccac accggcagaa gttacagtag actgtgaggc accccacctt 4560
   gctcctgatg cagtttctgt gccattgctg gtgctggtgg aggcagcgga gcagaggctc 4620
   aggcacaatg aagcgtggat gtgttctgca ggttgctgca aaactcacct tattctgact 4680
   tttggatttc atggcattcc aggaagctcc ttgccatgct gttggcctgg aagtccacct 4740
   gtctggtcca tagtgacgtc ctgaagagcc agtctgtaaa ataaccaacc acttacttag 4800
   cgtttggata gactccatgc cttctctctc cctgcaaaga aaaatcttga acatttatga 4860
   tgtcaattag tgaaagtatt gaaaatacta aattataact aaaagcaact ttttatgtta 4920
   ttgaaaatat tgaaagaact gatattaatg ataatcattt tatttttcat ctctctgata 4980
   tacccaatgt gtggcaatca gcccctacca cgagcattaa tgccatgtaa agctggcttt 5040
   ctggagtctg ccaggtccac acgaagtgct acccccagtt tctgctgtta cttgctgcct 5100
   ccaggccagg ggagcaggag atgctcagct ctggtgcctt ttcttttatt tcagtgctgc 5160
   cttcccaccc acccagtcca ttcaccctca cctctccctg catggaggca acagcatttc 5220
   aagatgtacc ttgggaagtc aggatagctg gaagtaaggg tttttgggat ccctgtggtc 5280
   tcttcactga tcatcgataa gcatttaaga gtgtgctaac accattcaca gaggtccctg 5340
   gaaaaaaata tataattttc tcacaaacaa ccaatacata acctatggca gttggttgaa 5400
   taattattga aaaaaagaac acgactgaaa aagttcatct gatgcctctg agcatgtgat 5460
   aaagtcctgg gtagacacgg aaacgtgttc ttacaaatga accttcggat ctatgaaaga 5520
   aaatatagta gggggactaa ggaacataga attttattag catatgtgat tttaccctta 5580
   tctttgtttc taatttaaag aaacaatttc agaaagtgtt agaagaattc tatgtttaat 5640
   aatgaatatt gttgagttca aaatattctc atatacccag atttacagag atgacacagt 5700
   attgaaatgg caggtgggct ctgtaagtta ttttggatta atgacattca gggtctttgc 5760
   agtggggact tcatgttgcc tctcactcat tcctcatatg tcagagtctt ctgtctatat 5820
   gcacatgctc aaagtccata ctcagtaggg gaaatctagc cggatggttc tccaattgtt 5880
   cctcggctgg attttcatca tcagatattt caactcatct catcagtttc ttgtaagaaa 5940
   aaaacatttt cactttgtag cacattttag ttatttttga gtcttcttcc ctaatagttt 6000
   gtaagcttaa agggacattt tattttccct ggtaaagagg attctaatat ttcaagaatg 6060
   tttcactgga attgatggga aatggtttct aaatggcatc gaagctggtc tttaatgagt 6120
   cttttgcaat gacttgggaa aaacaccctc cctcactaca ggatcttcat gttgttaata 6180
   atataaacaa acctttgaaa ttagcattgc aaaagaatcc catttttgtt tcgtaccaca 6240
   ctgttcaaag gaaaattgtt tatctctctt cctttctctc tctctcttat ttgacagaat 6300
   agcaagtgtt tagataattt cagatatatt ctaagtattt taccagctgt ggaataagtt 6360
   gtgtttgtcc atcactgtgt agctaccgtt cacatgttct ggctctgtac tccacgctta 6420
   tcgcgtgaac cctcacatgt tggactttca cagtgtgatc tctcacctgt cttggaaact 6480
   ccttcataaa gccgctcttc cttaggcctg ggctgtttgg aagtcctggt gaaactttgc 6540
   ggttcacatt ttaaattcct gaatgacctt ttcattctct ctttctttat ctttgttttt 6600
   gtccttcatt ccctcctttt gttcttcctt cctgccttct tttcttcttt cctttcttct 6660
   ttctttaatt cttcctttct tcctattttc ttggtacaag tggaacaaaa accaacacat 6720
   agttttaagc tacacctttc tgcacctgat gtaaaatgac attaatcact atttaacttc 6780
   taaaattatt tctgaataca gttggagata ggctggtttt catgaggaag ctggcttggc 6840
   tttagtctta catttaaatt ctttgaaagt ggctcccagt gctattcagg gtcctttctt 6900
   gaggccagac tcatcaccat ctactattga ttttacagtg cactgacagt ttacaggaag 6960
   gagaagaaca gaatttctgc acactacaca acatgtgggc ttcctgtagc tccagggaca 7020
   tgtagctttg gtgaaactgg ggttttgtaa cctctgaatg atatggactt agtgaattct 7080
   aaggaactca gggggcacgt ggtcaggctc caccgcacag aagagccaca gtctccagac 7140
   tcatggcgtt ccctccagaa ctccccattc ccctctgagc atatttctat gctgctgctt 7200
   cttgtgactt acatgaggca tctcagcttc ttcatgtttg tagggatggt tccctaagcc 7260
   tgttcaagta ggctctcact agagttacca ttcatatttg agaagaaaag aacatttaat 7320
   aaatgtatgt gtggggctga ggttctaaag gaattgaaaa gagacgataa acattgaatg 7380
   agggtgaggg catccctgct ggggagaaac ctctgtccct aggaagagtc cgttcatgtc 7440
   atgtggtttg ggatgaacca ggggtctggc cccatcgggt cacaggtgat ggcaaataga 7500
   aaagaagcaa atggaggaaa tagtcggata agtaggtgac gtgaaaggca ggacctggtc 7560
   accccagcaa gtgctatgga cagttcccgg aaacggttgc ccacttcaca ggtccatggg 7620
   tctgaccctt ggactctgcc aggatcaact gcccagagtg ccagagtttt agccaaaggt 7680
   gtacttactt ccttatttat ctgcaaaagg atggaaactg tgggagtcaa agcctatttt 7740
   gctgagtgtt cccactggag tctctggtag aattagcagg tcatgctgtc aaaatcatgg 7800
   acaaaggctg ggtgcagtgg ctcatgccta taatcccagc actttgggag gccaaggtgg 7860
   gcggatcacc tgagctcagg agtttgagac cagcctgggc aacatgggga aactccatct 7920
   ctacaaaata tacaaaatat tagccagcca tcgtggtgcg tgcctgtggt cccagtttct 7980
   tgggaggctg aggcgggagt atcatttgag ccaggaggtt gaggttgcag tgagctgaga 8040
   tcacatcact gcactccatc ctgggtgaca gagagagacc ctgtctcaaa aaaaaaaaaa 8100
   aagaacaaag tcttagcggg gggtctctat gtccccaagt ggctctctaa ggggatccat 8160
   tagcgtataa ttgaatatca aagggaattt taccttaaac atatatcaag tctatactca 8220
   gctaatcgtt ttgtctaaac acagccttgt tagcatttga atttacaaat attcatttgc 8280
   ttatttgcag tgttcctttt gcttttagaa taaatcataa atatttcccc aatactgtca 8340
   agatacttct tcaactttgt ccctgaacca tcagctttct ttagggtccg ggtatccttt 8400
   acacttcaat ggggttttct tgtgtgtgtc attgggggta actccattga cctcagcatt 8460
   tctatctcaa gagatggctc tggaacacag aaccagaggc aacaggtact aatggttgat 8520
   aaaattaaca gcgactgcat aaccattttc cacatggcag ggctgtgctt aagccccaca 8580
   cctccaataa tcaatttcta tgacggtact aataacctta actatatgca aattcttcac 8640
   cacttatgat atggtacaaa ctggagttta cagatggcct tggccttgcc tagcccattt 8700
   gttcatccta acttcttcat cacttcctga tgcacttggg gaagcatgtt gacacaacgt 8760
   taccaatatt tgattaccta tggttatctg caattaccta cgcttatctt atcagaccct 8820
   agatgccatt tgagcccctt acttctttgg gcagtgggtg aaatgggaga acatagagaa 8880
   atgttcactt tgacctaaga aacgcataaa agggaatgcc atgcacaatg taagataaaa 8940
   tgtttgggga caggggatca tcgtgaaatt tatttctgct attctgccct attgaggtca 9000
   ttaattataa tggatttaaa gatttttttt taaactctac aaacactaag ccaaggaatt 9060
   caggttatct gctatgttaa cctaataaag caaaggaaaa taagttgctt taatccttgc 9120
   ttgttaacct aataaagcaa aggaaaatat gttgctttaa tccttgcttc ttttctcttt 9180
   tggtaataga aaaggtaaat aagatgtaat gttgaactgc tggtgatgat ttctaaaacg 9240
   actctttaaa tactcctttc ctatggactt atattaaaga tgcattgtac acattgtttt 9300
   agcagataga tcaaagatat actcagtggt catctgttgc attatcagaa atttgataca 9360
   tgttacagaa gcatgaggaa gggaatccta tccacttttg aaaatagagt tcacattgca 9420
   agcgacatag ggcattatca aaaccaattc agagcctggc acaaaatgta tacttcctag 9480
   tgaaagctgt ctcaaaacac agcctttttg atggatcata ttagctcata tctgccatag 9540
   aaaggcaatc aagcttactc cctgcaatcg gagtcaatat caattcttgc tcaagcaaac 9600
   atgttttaca gtgttggcgc aatggcagga actagtcaag ggcactttca gatctggaaa 9660
   gaggaaagag gaggtagaaa atcactcttt tgtgtttctt aaactgtcaa tcaagcaaga 9720
   aaatttattc caccccttca tatttgctct aaaatctggg gccagtagtt gaaatgtgat 9780
   cttgttcttt ctcagccttt tggcaattta atgccaaata ttccaaatgt tagatgaatg 9840
   gcagtaataa aatgattcaa atgtttgata aatacaagct gagagcaaaa tctggactct 9900
   gaaaaatcgg aactatttta tcatgttgct aaaatgagag catcattttc ttccctctct 9960
   gtaagtgcgg tagtttaatt tcctagaaaa agttgctagt gccttgttta agatgatcat 10020
   ttatcatttc atgtggatat tattggtcta attagaagga gaagattgat tggatttact 10080
   ttaaagaaaa ttattctcct atgtctcttt gaactcagga atagaaaatg accaagaagc 10140
   acatcatctt cagatgtgat ttttgccctc actggggatg tagaaccaga ataggtacaa 10200
   catatgctca gaagagaggt gacttcagta ttagccttct agaaactaac atcactttaa 10260
   tttttacatg ttccaaaaaa aatcatagtg attgttctgt aataagaatg ttaacgtgat 10320
   gcctatagaa gttagaggtt tttgtttctg tttctcttca ggcaagtcca tagatagagg 10380
   aaaatcagac acaggctggt gacaactggt atagatacag tgagcatgga atgagaacca 10440
   cctacaacat tgcctgatta tttttaagtt tatatcaaaa ctgcttcaca agttcacaaa 10500
   agttgtcact cttaatttgg gttaagaaaa gtaatagtat tatctctgct gaagttgatt 10560
   gtttttctct tttaccatga ctgttggcca aaagcaagct gaacctctgc actttgaaac 10620
   aaagcacagt atgaatcttt aacccaagca cttagtttca ccactgagat tctacatcat 10680
   ctgcaagaaa atgcacaaac ttttggtcca atgttggatt cttcaaaagt gcatagcaaa    10740
   acttttgtgc tatgccgctt gatgtaactt ttaaagatgt tatcaaatgt taaatgcctc    10800
   attctgcgtt aatttgctgt tggctgtttt gtgaacacaa aataaacttg agattttttt    10860
   tttttttttt ttttgtgaaa ctcctccaaa gccaatttca gcccttggta agcttgcaga    10920
   cagttactga tcttaaattt aattttaaaa gacaatgtat cttaattata atttagcttt    10980
   ttaaaacaat gagatagctt tacatttccc ctttgtttga atgagaaaat ggatcttggg    11040
   ttgctatgct agaacacttg tagattgctg ggtcctttgt aagggggcca tggacacacc    11100
   acactttctt tcaatcctta catttgaagc attgatattc ttcaaaacct tcttgttaca    11160
   tgtgcgcaat agaaatttct aatgttcatg acttttatct ttcctgtcca tcaattcact    11220
   ggttgtaaat gcttcctgag agctgtctag gtctgtatcc cagattgttg cttaatgaca    11280
   tctgacagat gcattgtttt ctgaaatcag cttaagacac caattgtggc aactggaaac    11340
   tcattacctg ctgcattgga tcaactatgg aagttggagc aggggtgggc ggaggtcacc    11400
   taaccaatca atggaaggca actcacacct gctccaagcc tcagctttga gaaacaaaca    11460
   cgtttataag aaaaaatata tagctattat tacagaagtg aatatgttgt gctctcttac    11520
   tgctcttggt gcattgacag tttctgtatc tcaaccctat tcatctttat gaaaaagcat    11580
   tctgaagatc tatcctcagc actgctgagt gtgcagtcac actttcctac caaccccctt    11640
   cttaccatct ctagctgcca tttgtggggg gaacaaaaca agggaatcct gattgtgtac    11700
   agtataaatt gtattatatt ttttgtactt atgttttatg taaatagttt gtctcattca    11760
   attgtatgtg agcattgaaa taaatcctac catttagggg acaatttaaa aaaaaaaaaa    11820
   aaaaa                                                                11825
<210> 11
   <211> 1074
   <212> PRT
   <213> Human semaphorin 5A
<400> 11
   Met Lys Gly Thr Cys Val Ile Ala Trp Leu Phe Ser Ser Leu Gly Leu
   1               5                   10                  15
   Trp Arg Leu Ala His Pro Glu Ala Gln Gly Thr Thr Gln Cys Gln Arg
               20                  25                  30
   Thr Glu His Pro Val Ile Ser Tyr Lys Glu Ile Gly Pro Trp Leu Arg
           35                  40                  45
   Glu Phe Arg Ala Lys Asn Ala Val Asp Phe Ser Gln Leu Thr Phe Asp
       50                  55                  60
   Pro Gly Gln Lys Glu Leu Val Val Gly Ala Arg Asn Tyr Leu Phe Arg
   65                  70                  75                  80
   Leu Gln Leu Glu Asp Leu Ser Leu Ile Gln Ala Val Glu Trp Glu Cys
   85 90 95
   Asp Glu Ala Thr Lys Lys Ala Cys Tyr Ser Lys Gly Lys Ser Lys Glu
   100 105 110
   Glu Cys Gln Asn Tyr Ile Arg Val Leu Leu Val Gly Gly Asp Arg Leu
   115 120 125
   Phe Thr Cys Gly Thr Asn Ala Phe Thr Pro Val Cys Thr Asn Arg Ser
   130 135 140
   Leu Ser Asn Leu Thr Glu Ile His Asp Gln Ile Ser Gly Met Ala Arg
   145 150 155 160
   Cys Pro Tyr Ser Pro Gln His Asn Ser Thr Ala Leu Leu Thr Ala Gly
   165 170 175
   Gly Glu Leu Tyr Ala Ala Thr Ala Met Asp Phe Pro Gly Arg Asp Pro
   180 185 190
   Ala Ile Tyr Arg Ser Leu Gly Ile Leu Pro Pro Leu Arg Thr Ala Gln
   195 200 205
   Tyr Asn Ser Lys Trp Leu Asn Glu Pro Asn Phe Val Ser Ser Tyr Asp
   210 215 220
   Ile Gly Asn Phe Thr Tyr Phe Phe Phe Arg Glu Asn Ala Val Glu His
   225 230 235 240
   Asp Cys Gly Lys Thr Val Phe Ser Arg Ala Ala Arg Val Cys Lys Asn
   245 250 255
   Asp Ile Gly Gly Arg Phe Leu Leu Glu Asp Thr Trp Thr Thr Phe Met
   260 265 270
   Lys Ala Arg Leu Asn Cys Ser Arg Pro Gly Glu Val Pro Phe Tyr Tyr
   275 280 285
   Asn Glu Leu Gln Ser Thr Phe Phe Leu Pro Glu Leu Asp Leu Ile Tyr
   290 295 300
   Gly Ile Phe Thr Thr Asn Val Asn Ser Ile Ala Ala Ser Ala Val Cys
   305 310 315 320
   Val Phe Asn Leu Ser Ala Ile Ala Gln Ala Phe Ser Gly Pro Phe Lys
   325 330 335
   Tyr Gln Glu Asn Ser Arg Ser Ala Trp Leu Pro Tyr Pro Asn Pro Asn
   340 345 350
   Pro His Phe Gln Cys Gly Thr Val Asp Gln Gly Leu Tyr Val Asn Leu
   355 360 365
   Thr Glu Arg Asn Leu Gln Asp Ala Gln Lys Phe Ile Leu Met His Glu
       370                 375                 380
   Val Val Gln Pro Val Thr Thr Val Pro Ser Phe Met Glu Asp Asn Ser
   385                 390                 395                 400
   Arg Phe Ser His Val Ala Val Asp Val Val Gln Gly Arg Glu Ala Leu
                   405                 410                 415
   Val His Ile Ile Tyr Leu Ala Thr Asp Tyr Gly Thr Ile Lys Lys Val
               420                 425                 430
   Arg Val Pro Leu Asn Gln Thr Ser Ser Ser Cys Leu Leu Glu Glu Ile
           435                 440                 445
   Glu Leu Phe Pro Glu Arg Arg Arg Glu Pro Ile Arg Ser Leu Gln Ile
       450                 455                 460
   Leu His Ser Gln Ser Val Leu Phe Val Gly Leu Arg Glu His Val Val
   465                 470                 475                 480
   Lys Ile Pro Leu Lys Arg Cys Gln Phe Tyr Arg Thr Arg Ser Thr Cys
                   485                 490                 495
   Ile Gly Ala Gln Asp Pro Tyr Cys Gly Trp Asp Val Val Met Lys Lys
               500                 505                 510
   Cys Thr Ser Leu Glu Glu Ser Leu Ser Met Thr Gln Trp Glu Gln Ser
           515                 520                 525
   Ile Ser Ala Cys Pro Thr Arg Asn Leu Thr Val Asp Gly His Phe Gly
       530                 535                 540
   Val Trp Ser Pro Trp Thr Pro Cys Thr His Thr Asp Gly Ser Ala Val
   545                 550                 555                 560
   Gly Ser Cys Leu Cys Arg Thr Arg Ser Cys Asp Ser Pro Ala Pro Gln
                   565                 570                 575
   Cys Gly Gly Trp Gln Cys Glu Gly Pro Gly Met Glu Ile Ala Asn Cys
               580                 585                 590
   Ser Arg Asn Gly Gly Trp Thr Pro Trp Thr Ser Trp Ser Pro Cys Ser
           595                 600                 605
   Thr Thr Cys Gly Ile Gly Phe Gln Val Arg Gln Arg Ser Cys Ser Asn
       610                 615                 620
   Pro Thr Pro Arg His Gly Gly Arg Val Cys Val Gly Gln Asn Arg Glu
   625                 630                 635                 640
   Glu Arg Tyr Cys Asn Glu His Leu Leu Cys Pro Pro His Met Phe Trp
                   645                 650                 655
   Thr Gly Trp Gly Pro Trp Glu Arg Cys Thr Ala Gln Cys Gly Gly Gly
               660                 665                 670
   Ile Gln Ala Arg Arg Arg Ile Cys Glu Asn Gly Pro Asp Cys Ala Gly
           675                 680                 685
   Cys Asn Val Glu Tyr Gln Ser Cys Asn Thr Asn Pro Cys Pro Glu Leu
       690                 695                 700
   Lys Lys Thr Thr Pro Trp Thr Pro Trp Thr Pro Val Asn Ile Ser Asp
   705                 710                 715                 720
   Asn Gly Gly His Tyr Glu Gln Arg Phe Arg Tyr Thr Cys Lys Ala Arg
                   725                 730                 735
   Leu Ala Asp Pro Asn Leu Leu Glu Val Gly Arg Gln Arg Ile Glu Met
               740                 745                 750
   Arg Tyr Cys Ser Ser Asp Gly Thr Ser Gly Cys Ser Thr Asp Gly Leu
           755                 760                 765
   Ser Gly Asp Phe Leu Arg Ala Gly Arg Tyr Ser Ala His Thr Val Asn
       770                 775                 780
   Gly Ala Trp Ser Ala Trp Thr Ser Trp Ser Gln Cys Ser Arg Asp Cys
   785                 790                 795                 800
   Ser Arg Gly Ile Arg Asn Arg Lys Arg Val Cys Asn Asn Pro Glu Pro
                   805                 810                 815
   Lys Tyr Gly Gly Met Pro Cys Leu Gly Pro Ser Leu Glu Tyr Gln Glu
               820                 825                 830
   Cys Asn Ile Leu Pro Cys Pro Val Asp Gly Val Trp Ser Cys Trp Ser
           835                 840                 845
   Pro Trp Thr Lys Cys Ser Ala Thr Cys Gly Gly Gly His Tyr Met Arg
       850                 855                 860
   Thr Arg Ser Cys Ser Asn Pro Ala Pro Ala Tyr Gly Gly Asp Ile Cys
   865                 870                 875                 880
   Leu Gly Leu His Thr Glu Glu Ala Leu Cys Asn Thr Gln Pro Cys Pro
                   885                 890                 895
   Glu Ser Trp Ser Glu Trp Ser Asp Trp Ser Glu Cys Glu Ala Ser Gly
               900                 905                 910
   Val Gln Val Arg Ala Arg Gln Cys Ile Leu Leu Phe Pro Met Gly Ser
           915                 920                 925
   Gln Cys Ser Gly Asn Thr Thr Glu Ser Arg Pro Cys Val Phe Asp Ser
       930                 935                 940
   Asn Phe Ile Pro Glu Val Ser Val Ala Arg Ser Ser Ser Val Glu Glu
   945                 950                 955                 960
   Lys Arg Cys Gly Glu Phe Asn Met Phe His Met Ile Ala Val Gly Leu
                   965                 970                 975
   Ser Ser Ser Ile Leu Gly Cys Leu Leu Thr Leu Leu Val Tyr Thr Tyr
               980                 985                 990
   Cys Gln Arg Tyr Gln Gln Gln Ser  His Asp Ala Thr Val  Ile His Pro
           995                 1000                 1005
   Val Ser  Pro Ala Pro Leu Asn  Thr Ser Ile Thr Asn  His Ile Asn
       1010                 1015                 1020
   Lys Leu  Asp Lys Tyr Asp Ser  Val Glu Ala Ile Lys  Ala Phe Asn
       1025                 1030                 1035
   Lys Asn  Asn Leu Ile Leu Glu  Glu Arg Asn Lys Tyr  Phe Asn Pro
       1040                 1045                 1050
   His Leu  Thr Gly Lys Thr Tyr  Ser Asn Ala Tyr Phe  Thr Asp Leu
       1055                 1060                 1065
   Asn Asn  Tyr Asp Glu Tyr
       1070

## Description

The present invention relates to a diagnostic method for the determination of GvHD or HvGD of the immune system, especially of Natural Killer (NK)-cells and of T-cells, using the determination of presence of a blood: peripheral mononuclear cell or plasma marker. The marker can be detected directly, e.g. by determination of its titre using a specific antibody against the marker, or indirectly, e.g. by the determination of the mRNA encoding the marker.

Further, the invention relates to the use of the protein corresponding to the marker analysed in the analytical method, as a medicament for activating the cytotoxicity of NK cells as well as for activating the cytotoxicity and the proliferation of T-cells.

The diagnostic method is especially suitable for use in the determination of the status of the immune system in samples obtained from patients having received a transplant, which especially is an allogeneic transplant of stem cells, e.g. of bone marrow stem cells in order to determine the state of the graft-versus-host (GvH) disease. Further, the diagnostic method is suitable for determination of the status of the immune system in a sample obtained from a patient having an autoimmune disease, or for monitoring the state of activity of the immune system in a sample obtained from a patient who is receiving medication influencing the activity of the immune system.

In a second aspect, the invention especially relates to the activation of the immune system by making use of the characteristics of the protein to activate the immune response, e.g. in an immuno-compromised patient. Therein, the invention relates to the compound for use as a medicament or for use in the manufacture of a medicament, especially for the treatment of a low activity of the immune system. As a medicament, Semaphorin 5A protein is preferably contained in a pharmaceutically acceptable formulation, e.g. for i.v. administration to a patient.

### State of the art

Generally, for diagnosis of the GvH disease, which is indicated e.g. by skin rashes, an organ biopsy is generally used.

Paczesny et al. (Science Translational Medicine 2, 13ra2 (2010)) described the analysis of Elafin as a specific biomarker in plasma for the graft versus host disease.

US 2005/042636 A1 describes that abnormally proliferating cells, i.e. tumour cells, overexpress the Sema5 gene product and accordingly SEMA5 antigens can be used to generate an immune response against tumour cells, e.g. SEMA5 specific cytolytic responses and protective antibodies against SEMA5 as well as Sema5 antisense nucleic acids to inhibit expression of Sema5. For SEMA5A, cell membrane localization was verified.

WO2005/104785 A2 describes a medicament for the treatment of liver cancer containing as the active agent an antibody directed against a membrane-associated peptide, e.g. prostaglandin receptor membrane component 1, a membrane-bound transporter (SLC2A2), or SEMA5A, as these peptides have been found to be up-regulated in tumour tissue compared to healthy tissue.

The state of art uses SEMA5A as a tumour marker or as an anticancer target, respectively, but not for determining the activity of the immune system.

Generally, it is known that tumours necessarily evade being recognized by the immune system, i.e. tumours are not recognized as such by the immune system, and therefore tumours naturally do not raise an immune response.

### Objects of the invention

It is an object of the present invention to provide for an alternative diagnostic test method that can reliably determine the state of the immune system, e.g. a state of the immune response indicative of the GvH disease, transplant rejection, including organ rejection, and indicative for the HvG reaction (HvGD), or indicative of an autoimmune disease. A further object is to provide a compound for use in the activation of the immune system.

### General description of the invention

The invention achieves the above-mentioned objects by the features defined in the claims, and especially by providing an analytical or diagnostic method, and an analytical or diagnostic test kit suitable for performing the method, which analyses the concentration of Semaphorin 5A, either of Semaphorin 5A (SEMA5A) protein or peptide, e.g. by detecting the reaction of an antibody having specificity for SEMA5A in a human patient sample, and/or of Semaphorin 5A-specific mRNA. In this first embodiment of the invention, there is provided a method for determining GvHD or HvGD by determining the concentration of Semaphorin 5A, either by determining the concentration of Semaphorin 5A protein, and/or by the determining the concentration of Semaphorin 5A-specific mRNA, wherein the concentrations of Semaphorin 5A protein or mRNA are determined in relation to the concentration of Semaphorin 5A protein or mRNA determined in healthy humans, and/or in relation to the concentration of a housekeeping gene product, i.e. protein, or mRNA thereof. The concentration of Semaphorin 5A in the patient sample, preferably also in a sample of a healthy human, is determined in relation to a constitutively expressed component of the sample, e.g. the gene product of a house-keeping gene for normalization, and/or the concentration of Semaphorin 5A in the patient sample is determined in relation to the concentration of Semaphorin 5A that is determined in at least one sample of at least one healthy human. The method further comprises the step of correlating the concentration of Semaphorin 5A determined in the patient sample to the concentration of Semaphorin 5A determined in the sample of at least one healthy human, and the step of assigning an elevated concentration of concentration of Semaphorin 5A in the patient sample over the concentration of Semaphorin 5A in the sample of the at least one healthy human to GvHD or HvGD of the patient.

In a second embodiment, the present invention provides Semaphorin 5A for use as a pharmaceutical active agent of a medicament in the treatment of a lack of immune activity, i.e. for increasing the proliferative and cytotoxic activity of NK cells, and for increasing the proliferation and the cytotoxic activity of T-cells, especially CD8⁺-T-cells. In greater detail, Semaphorin 5A is provided for use as a medicament for increasing the cytotoxicity of NK cells, and increasing the proliferation of T-cells, especially CD8⁺-T-cells. Preferably, Semaphorin 5A is for use as a medicament in combination with IL-2 and/or IL-15. Accordingly, this embodiment also relates to a medicament comprising Semaphorin 5A which is adapted or prepared for the medical treatment.

The patients from which the samples originate that are tested for the titre of SEMA5A, and the patients receiving SEMA5A as a medicament preferably are transplant patients and patients having been diagnosed or being suspected of having an autoimmune disease, and preferably excluding tumour patients. Preferred transplant patients are recipients of one of the group comprising or consisting of allogeneic hematopoietic stem cells, solid organs or tissue, e.g. kidney, liver, heart, lung, skin. Preferred autoimmune diseases are comprised in the group containing or consisting of lupus erythematodes and rheumatoid arthritis.

The embodiments of the invention are based on the observation that the titre of Semaphorin 5A and of Semaphorin 5A-mRNA is increased in patients being suspected of GvH disease or having clinically manifest GvH disease by a factor of about 20 in comparison to healthy patients. Further, it has been observed that the immune system can be activated by administration of Semaphorin 5A protein, as e.g. exemplified by the *in vitro* activation of the cytotoxic activity and increased cytotoxic activity of NK cells by Semaphorin 5A, and as exemplified by the proliferation of CD8⁺ T-cells upon addition of the recombinantly produced Semaphorin 5A protein to the cultivation medium. The activation of NK cells by presence of SEMA5A could also be shown by induction of cytokine secretion upon stimulation ofNK cells with Sema5A in comparison to the cytokine secretion profile of non-stimulation NK cells, e.g. induction of increased secretion of IL-1β, IL-6, and 1L-8. Furthermore, a significantly increase in the secretion of IFN-γ, IL-1β; IL-6, IL-8, TNF-α, GM-CSF was measured when NK cells were incubated in the presence of SEMA5A in combination with IL-2 and IL-15 in comparison to the cytokine levels detected in the presence of this cytokine combination alone.

It was found that in patients having received a transplant, e.g. an allogeneic kidney or allogeneic hematopoietic stem cells suffering from acute transplant rejection or GvHD, respectively, and in patients having an autoimmune disease, e.g. having been diagnosed with rheumatoid arthritis, an increased concentration of Semaphorin 5A, determined both as protein and mRNA, correlates with the disease. In patients who suffered both from chronic GvHD and from cancer, e.g. thyroid or lung cancer, and who underwent anti-cancer therapy the correlation of the increase in the concentration of SEMA5A to the GvHD or autoimmune disease was found to a smaller extent. Accordingly, the analytical method of the invention preferably indicates the presence of an acute GvHD or HvGD, and an acute phase of an autoimmune disease.

Semaphorin 5A as the analyte of the method of analysis is not necessarily bound to cells, but can also be determined in cell-free samples, e.g. in blood serum. The sample used in the analytical method can therefore be selected from blood, including whole blood, blood cells, e.g. PBMC, and preferably the sample is serum, i.e. the cell-free component of blood.

### Detailed description of the invention

The invention will now be described in greater detail with reference to the figures, wherein
- Figure 1 shows mRNA levels of Semaphorin 5A in PBMCs in allogeneic hematopoietic stem cell transplant recipients,
- Figure 2 shows mRNA levels of Semaphorin 3A in hematopoietic stem cell therapy patients,
- Figure 3 shows a Western blot for Semaphorin5A protein in patient sera,
- Figure 4 shows concentrations of Semaphorin 5A protein in patients prior to and after hematopoietic stem cell transplantation,
- Figure 5 shows concentrations of mRNA of Semaphorin 5A, normalized to β-actin mRNA levels, in healthy humans and in patients after hematopoietic stem cell transplantation,
- Figure 6 shows concentrations of Semaphorin 5A protein in healthy humans and in patients after kidney transplantation,
- Figure 7 shows concentrations of Semaphorin 5A protein in patients diagnosed with rheumatoid arthritis and in healthy humans,
- Figure 8 shows concentrations of Semaphorin 5A mRNA in patients diagnosed with rheumatoid arthritis and in healthy humans,
- Figure 9 shows the recombinant Semaphorin5A detected by Western Blot after purification by affinity chromatography.,
- Figure 10 shows the upregulation of the activation marker NKp44 NK cells upon stimulation with Semaphorin 5A,
- Figure 11 shows the upregulation of activation marker CD25 on NK cells upon stimulation with Semaphorin 5A,
- Figure 12 shows the expression levels of NKG2A and NKG2D receptors on NK cells upon stimulation with Semaphorin 5A,
- Figure 13 shows the upregulation of Granzyme B mRNA in NK cells induced by Semaphorin 5A,
- Figure 14 shows the effect of Semaphorin 5A in increasing the cytotoxic potential of NK cells against target cells,
- Figure 15 shows the effect of Semaphorin 5A on the proliferation of CD8⁺ T-cells, and
- Figure 16 shows the upregulation of Granzyme B in CD8⁺ T-cells by Semaphorin 5A.

In the figures, p<0.05 is indicated by *, and p<0.01 is indicated by **, which is both considered as statistically significant in the Mann-Withney U-test applied in the statistical analysis. In the figures, the same abbreviations denote the same samples or experiments, respectively.

For identifying a marker that is suitable for identifying a status of activity of the immune system indicating GvH disease, approximately 60 patients having received hematopoietic stem cell therapy (HSCT) using allogeneic stem cells were monitored for the GvH disease (GvHD), i.e. the activation of the immune cells was monitored. In accordance with standard practice, this was done by a biopsy, because no validated diagnostic blood test is available that can determine the graft versus host disease, i.e. an activation of the immune system in response to antigen.

When searching for a biomarker that is indicative of the activation of the immune system or occurring in the GvH disease, it has been found that the level of Semaphorin 5A protein in peripheral blood is a reliable marker for the presence of an increased activity of the immune system, i.e. for the immune response of the graft versus host disease in the transplant patients.

It has been found that levels of Semaphorin 5A, e.g. determined as Semaphorin 5A mRNA, showing an increase by a factor of at least 2, preferably by a factor of at least 4 to 5, preferably by a factor of at least 10, more preferably by a factor of about 15, and most preferably by a factor of about 20 or above in relation to an average concentration of Semaphorin 5A indicate an increased activity of the immune system, which is e. g. indicative of GvH disease in hematopoietic stem cell transplant recipients, or of an autoimmune disease. Accordingly, the diagnostic method preferably includes the step of normalizing the signal specifically detected for Semaphorin 5A to a constitutively expressed gene product, and determining the relation of the normalized signal for Semaphorin 5A to the signal for Semaphorin 5A which has been determined and normalized in a sample obtained from a healthy person. The resulting relation of the signal for Semaphorin 5A of the patient to the signal obtained in a healthy person can be edited, e.g. for subsequent medical evaluation.

Generally, Semaphorin 5A, or the mRNA encoding Semaphorin 5A, can be analysed by the method in a sample obtained from a patient, which sample is preferably a cell-containing sample, e.g. blood, and specifically peripheral blood mononuclear cells (PBMC).

Preferably, the concentrations of Semaphorin 5A are standardized by relating them to the signal determined for a constitutive sample component. For embodiments of the method for analysis determining the concentration of Semaphorin 5A by RT-PCR, i.e. by reverse transcription of RNA isolated from the sample to cDNA, followed by specific amplification of a fragment of the semaphorin 5A coding sequence by PCR, the β-actin encoding sequence can be the constitutive sample component, in relation to which the signal specific for Semaphorin 5A is normalized.

As an alternative to determining the mRNA by reverse transcription of RNA to produce cDNA, followed by PCR on the basis of the cDNA using primers specific for the semaphorin 5A coding sequence, a specific antibody reaction against Semaphorin 5A can be used. In this embodiment, cell-free serum can be used for the analysis of Semaphorin 5A, especially for the determination of Semaphorin 5A protein, e.g. using an antibody having specificity to Semaphorin 5A protein in an ELISA. Polyclonal, preferably monoclonal antibody that is specifically directed against Semaphorin 5A can be produced by standard methods, e.g. by immunizing an experimental animal with Semaphorin 5A and collecting serum for polyclonal antibody, or using spleen cells from the immunized animal for generation of hybridoma and selecting a clone to produce a monoclonal antibody.

When analysing the activity of Semaphorin 5A (protein) on immune cells in *in vitro* assays, it has been found that the addition of Semaphorin 5A to the cultivation medium increases the cytotoxic activity of NK cells, the cytotoxic activity of CD8⁺ T-cells, and the proliferation of CD8⁺ T-cells, also in the absence of target cells, e.g. of allogeneic cells. Accordingly, in the second embodiment, the invention provides Semaphorin 5A in a pharmaceutical composition or as an ingredient in a medicament for use in the activation of the immune system, especially for activating the cytotoxic activity of NK cells, for activating the cytotoxic activity of CD8⁺ T-cells, and for the proliferation of CD8⁺ T-cells. Preferably, the use of Semaphorin 5A is in the treatment of an insufficient immune system. The use of Semaphorin 5A as a medicament for the treatment of a low activity of the immune system, and Semaphorin 5A for use in the preparation of a pharmaceutical composition for use in the treatment of a low activity of the immune system, e.g. for increasing the activity of the immune system, preferably in patients suffering from congenital or aquired immune deficiencies, infectious diseases or cancer.

More preferably, the Semaphorin 5A for use as a medicament is a combination of Semaphorin 5A with IL-2 and/or with IL-15 for use as a medicament, especially for activating the immune system.

### Example 1: Semaphorin 5A is specifically increased in GvHD patients and HvGD patients

After written consent, as applicable, peripheral blood mononuclear cell samples were isolated from 60 patients (30-60 years old) who underwent allogeneic hematopoietic stem cell therapy. Prior to this therapy, similar conditioning regimens were applied. From these patients, 5 were suspected of GvH disease, 6 patients presented clinical signs of GvH disease at the time the analysis was made, or had been diagnosed with GvH disease up to two months before, and a further 6 patients had been diagnosed with clinical GvH disease at 6-2 months before the analysis was made. 43 of the patients never developed GvH disease or were free of GvH disease for more then 6 months. As a control, PBMCs from 10 healthy individuals was used.

Total RNA was isolated from PBMC samples, and reverse transcription was performed on RNA to produce complementary DNA (cDNA) using a high capacity cDNA reverse transcription kit (Applied Biosystems, Foster City, USA). For each reaction, 50 ng total RNA was used. Semaphorin 5A and, in parallel reactions, Semaphorin 3A and β-actin mRNA were determined using a two-step real time PCR (TaqMan Gene Expression Master Mix, Applied Biosystems, Foster City, USA) using amplification primers and FAM-labelled minor groove binding (MGB) Taqman probes.

For Semaphorin 5A, the primers corresponding to SEQ ID NO 1 (5'-CAACGGCGGCCACTATG -3') and to SEQ ID NO 2 (5'- CCAGGCGGGCTTTGC -3'), using MGB-probe of SEQ ID NO 3 (5'FAM- CAACGATTCCGATACACA 3') were used, for Semaphorin 3A primers having SEQ ID NO 4 (5'- TTCAGCAATGGAGCTTTCCA -3') and SEQ ID NO 5 (5'- CAACCCCAGCCGTTGAAC -3') with the MGB-probe according to SEQ ID NO 6 (5'-FAM- AAGCAGCAACAACTATATA -3') were used, and for β-actin primers according to SEQ ID NO 7 (5'- ATG ATG ATA TCG CCG CGC -3') and SEQ ID NO 8 (5'- GCC TTG CAC ATG CCG G -3'), and MGB-probe having SEQ ID NO 9 (5'-FAM- CGT CGT CGA CAA CGG -3') were used. For standardizing levels of Semaphorin 5A and of Semaphorin 3A mRNAs, respectively, the specific amount of β-actin-mRNA of parallel aliquots was used. Thermal cycling in a Step One plus real-time PCR system (Applied Biosystems) was 95°C for 10min, followed by 40 cycles at 95°C for 15s and 60°C for 1min. For analysis, the Step One software version 2.0 (Applied BioSystems) and a quantification-comparative method were used. Generally, patient data for Semaphorin 5A were normalized to the Semaphorin 5A signals of the healthy control group after normalizing each signal of Semaphorin 5A to that of β-actin.

The mRNA for human Semaphorin 5A is available under accession number NM_003966.2 from GenBank (SEQ ID NO 10). Preferably, the nucleotide sequence of Semaphorin 5A corresponds to SEQ ID NO 10, and the specific identification of Semaphorin 5A uses a nucleotide sequence according to SEQ ID NO 10, e.g. contained in a sample and used in the above described RT-PCR. Further, Semaphorin 5A protein according to this invention is preferably encoded by nucleotide sequence according SEQ ID NO 10, more preferably from nt 779 to nt 3934, and most preferably, Semaphorin 5A protein according to this invention has an amino acid sequence according to SEQ ID NO 11.

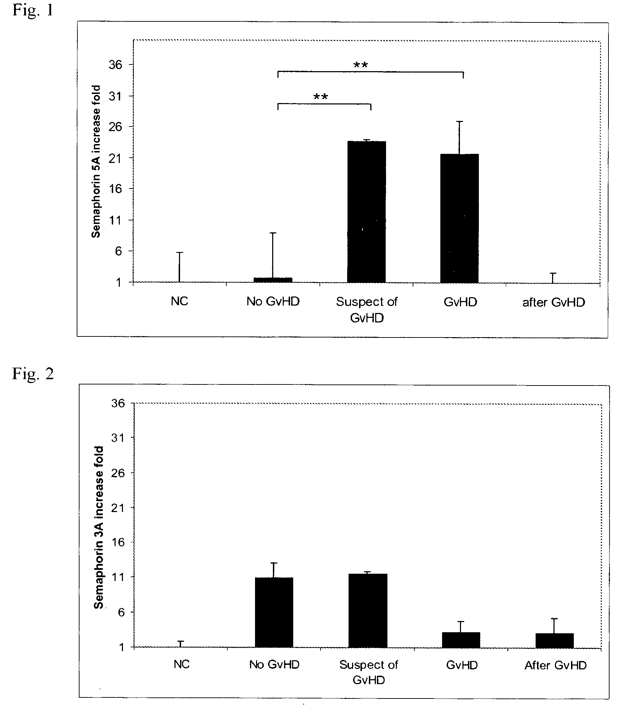

It was found that in comparison to the group without GvH disease (relative quantification (RQ):1.7±7.2), the patients suspected of GvH disease (RQ: 23.7±0.33; p<0.001) and patients with clinical diagnosis of GvH disease (RQ: 21.7±5.3; p<0.001) showed a significant increase of mRNA levels specific for Semaphorin 5A. After hematopoietic stem cell transplantation, no significant difference in the levels of Semaphorin 5A mRNA was observed in the group of patients after GvH disease (RQ: 1±1.7), when compared to the group without GvH disease. Results are shown in Figure 1, wherein mRNA levels of Semaphorin 5A are normalized to β-actin mRNA levels and the levels of Semaphorin 5A mRNA are normalized to the values of the healthy control group. The data show that the level of Semaphorin 5A in PBMCs is indicative of the increased activity of the immune system occurring in GvH disease. In detail, the group of patients which are suspected of GvH disease (Suspect of GvH) and the group of patients being diagnosed with GvH disease (GvHD) have an increase in the normalized mRNA levels of Semaphorin 5A by a factor of about 20 in comparison to the control group (NC), and in relation to the patients without GvH disease (No GvHD). After treatment of the patients suffering from GvH disease (after GvHD), i.e. by standard immunosuppressant therapy, levels of Semaphorin 5A were in the same range as those of healthy control and no GvHD-patients, demonstrating the diagnostic value of Semaphorin 5A levels for GvH disease.

The analysis of Semaphorin 3A in the same patient samples showed that Semaphorin 5A was upregulated in GvH disease-cases, whereas Semaphorin 3A was not. In detail, for Semaphorin 3A, no difference was measured between patients without GvH disease (No GvHD, RQ: 10.9±2.25) and patients suspected of GvH disease (Suspect of GvHD, RQ: 11.5± 0.4), and no differences were observed in patients diagnosed with GvH disease (GvHD, RQ: 3.2±1.59) and patients after GvH disease (After GvHD, RQ: 3.1±2.1), which results are shown in greater detail in Fig. 2.

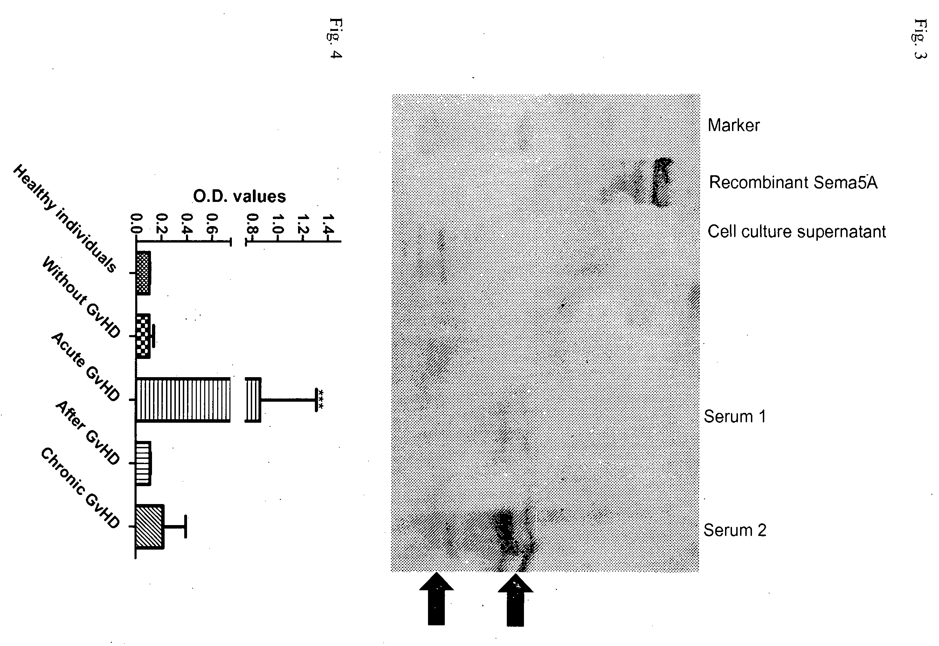

SEMA5A, i.e. Semaphorin 5A protein, was determined in an ELISA using a rabbit antibody that is specific for the N-terminal region of SEMA5A (available from Abcam), and an anti-rabbit antibody labelled by coupling to horse-raddish peroxidase (HRP) as a secondary antibody. A Western blot analysis using the antibody (Abcam) specific for SEMA5A revealed that a soluble form of SEMA5A is present in sera of patients having GvHD and rheumatoid arthritis. The Western blot, done by SDS-PAGE and electrotransfer onto a nitrocellulose membrane according to standard procedures is shown in Fig. 3. For control, recombinant Semaphorin 5A protein, cell culture supernatant from cultivated HEK293 cells overexpressing human SEMA5A were used (3 lanes from the left, one empty lane), a serum from a rheumatoid arthritis patient (Serum 1), and a serum from a GvHD patient (Serum 2). As both patients were free from cancer, this analysis shows that the soluble form of SEMA5A can be detected in sera of patients having an undesired immune response, which is exemplified by GvHD and the autoimmune disease.

Sera were collected from a group of patients (n=27) prior to transplantation (HSCT), without GvHD after transplantation (n=24), for suspect or confirmed GvHD (n=3) after GvHD episodes (n=3), or from patients transplanted for more than 3 years with chronic GvHD (n=10). Sera from 10 healthy individuals served as a control. Results are shown as OD of the ELISA in Figure 4, indicating a significant increase of SEMA5A serum concentration in patients with suspected or diagnosed acute GvHD (0.867±0.446; p<0.001) in comparison to the concentrations determined in the sera of patients after HSCT who were free of GvHD (0.106±0.003), or in sera from those HSCT patients after being treated for GvHD (0.114±0.005). These results clearly indicate that SEMA5A concentrations are upregulated specifically in case of acute GvHD. A separate group of patients suffering of chronic GvHD was analysed. A non-significant increase of SEMA5A concentrations was observed (0.219±0.178). However, the chronic patients presented other severe malignancies such as lung or thyroid cancer and were under therapy at the time the sample was taken. A correlation of the SEMA5A concentrations from these patients with GvHD would therefore be hindered by the impact of anti-cancer therapy.

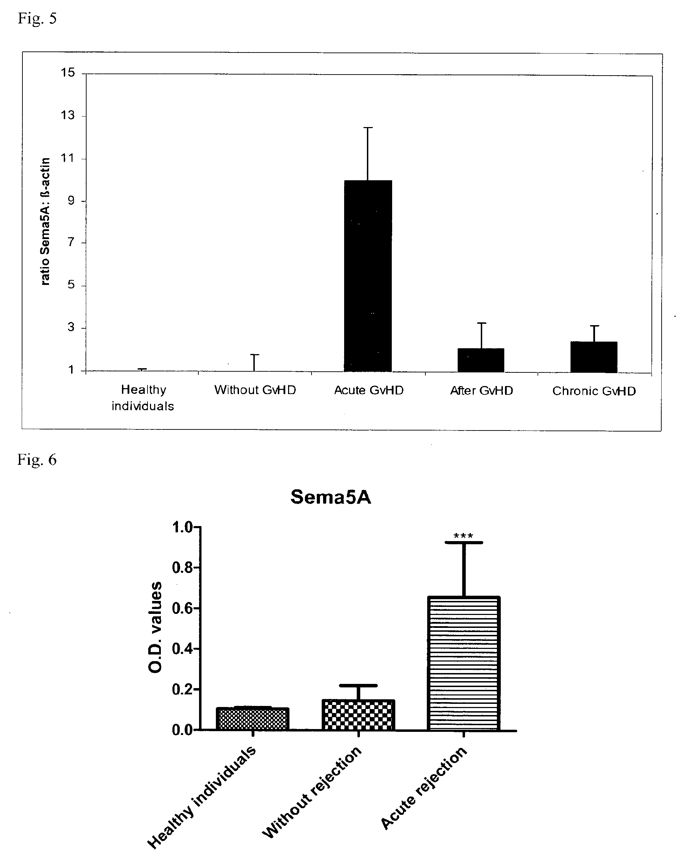

In addition, concentrations of mRNA encoding Semaphorin 5A (Sema5A mRNA) were detected by real-time PCR as described above. As shown for SEMA5A, elevated concentrations in the Sema5A mRNA in patients correlate with suspect or diagnosed GvHD (RQ 10±2.5, p<0.05) in comparison to Sema5A mRNA concentrations in patients after HSCT without GvHD (RQ 1±0.8) or after GvHD treatment (RQ 2.1±1.2). The group of patients showing chronic GvHD showed a RQ mean of 2.4±0.8. The results are shown in Figure 5.

As a further example of transplant recipients, in which elevated levels of Sema5A mRNA or SEMA5A indicate an undesired immune response, e.g. the host versus graft disease (HvGD), samples from patients having received an allogeneic kidney transplant were analysed. Results are shown in Figure 6 as OD obtained in the ELISA specific for SEMA5A, wherein *** indicates a significance p<0.001 in the one-way ANOVA statistical test followed by the post-hoc Bonferroni test. Sera from healthy individuals were used as control. This analysis makes it clear that also in the situation of solid organ transplantations, elevated concentrations of Semaphorin 5A indicate an increased activation of the immune system of the transplant recipient, i.e. HvGD, whereas controls and sera from transplant recipients who were free from HvGD, i.e. acute transplant rejection (Acute rejection) were analysed to have significantly lower serum concentrations of SEMA5A.

### Example 2: Semaphorin 5A is specifically increased in patients having an autoimmune disease

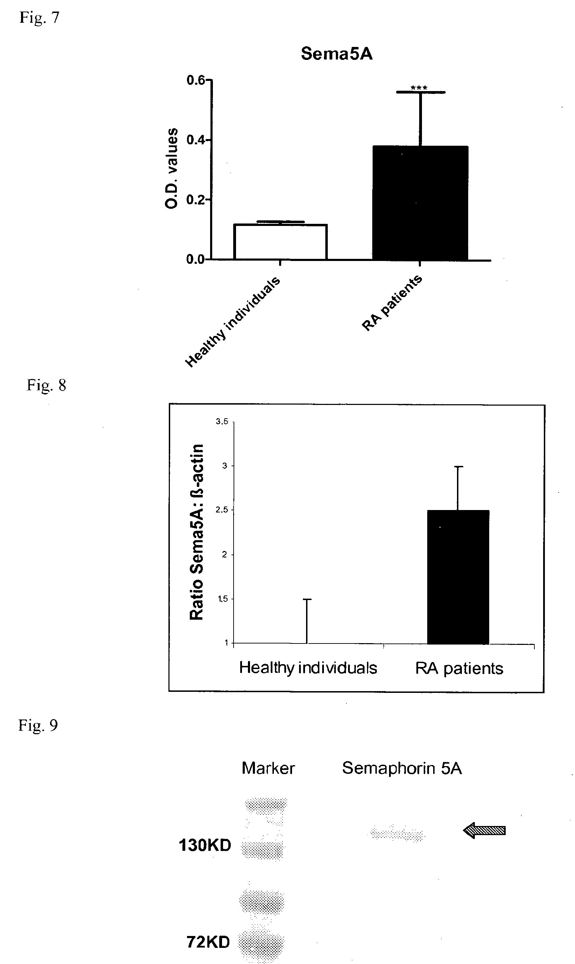

As an example for autoimmune diseases, patients who were diagnosed with rheumatoid arthritis were used. In detail, SEMA5A concentrations were assessed by ELISA in sera from 15 rheumatoid arthritis (RA) patients prior any kind of therapy, and in 10 healthy individuals. Significantly increased SEMA5A concentrations were detected in RA patients (OD 0.380±0.182, p<0.001) compared to the levels observed in healthy individuals (OD 0.117±0.009). The results are shown in Figure 7, wherein *** indicates a significance p<0.001 in the one-way ANOVA statistical test followed by the post-hoc Bonferroni test.

Determination of Sema5A mRNA concentrations in PBMCs enriched from whole blood of patients suffering of rheumatoid arthritis showed a significantly higher Sema5A mRNA concentration (RQ 2.5±1.2) in comparison to the concentrations measured in healthy individuals. Results are shown in Figure 8, wherein Sema5A mRNA concentrations are normalized to the concentration of mRNA of the housekeeping gene β-actin, which was detected in parallel by quantitative RT-PCR.

### Example 3: Production of recombinant Semaphorin 5A

As an example for the manufacture of Semaphorin 5A, e.g. for use in the production of a pharmaceutical composition containing Semaphorin 5A for use in the treatment of a low activity of the immune system, the extracellular domain of human Semaphorin 5A was expressed in HEK cells. For ease of purification, the expression construct included a His-tag. Briefly, the plasmid pcDNA3.1 was provided with the coding sequence for the extracellular domain of human Semaphorin 5A with an added soluble His-tag, and transfected using lipofection (Lipofectamine 2000, Invitrogen) into the human embryonic kidney 293 (HEK293) cell line. The recombinant Semaphorin 5A protein with its added His-tag was isolated from cell culture supernatant by immobilized-metal affinity chromatography on HisTrapFF columns (GE Healthcare, Little Chalfont, Great Britain). Cell culture supernatants were pooled, adjusted to pH 8.0, and loaded onto the columns using a BioLogic DuoFlow System, Bio-Rad, Hercules, USA). Elution of Semaphorin 5A was by 20 mM sodium phosphate, 0.5 M sodium chlorate, 0.5 M imidazole, pH 7.4. Recombinant protein was quality-controlled by Western blotting and an ELISA using detection antibodies against the V5 epitope and the His-tag (available from Invitrogen). The result is shown in Fig. 9, with the arrow indicating Semaphorin 5A.

### Example 4: Production of an antibody having specificity against Semaphorin 5A

Polyclonal and monoclonal antibody specific for Semaphorin 5A was produced according to standard practice in experimental animals. In short, experimental mice were vaccinated with Semaphorin 5A as produced according to example 2, using at least 2 booster vaccinations. For polyclonal antibody, serum was collected and purified as generally known. The specificity could be determined against immobilized Semaphorin 5A by detecting bound antibody using a secondary antibody.

For monoclonal antibody, spleen cells of a mouse were used for the hybridoma technique, and from hybridoma cultures, a producer was screened according to the affinity of the supernatant to immobilized Semaphorin 5A.

### Example 5: Activation of NK cells and of the cytotoxic activity of NK cells by Semaphorin 5A protein

Natural killer cells (NK cells) were isolated from peripheral blood of 8 healthy donors using magnetic assisted cell separation technology. Cells were cultured in the presence of IL-2 and IL-15, in the presence of Semaphorin 5A alone, or in a combination of Semaphorin 5A with IL-2 and IL-15. As a control, heat-denatured Semaphorin 5A was used. Cultivation was generally in standard cell culture medium under standard cultivation conditions.

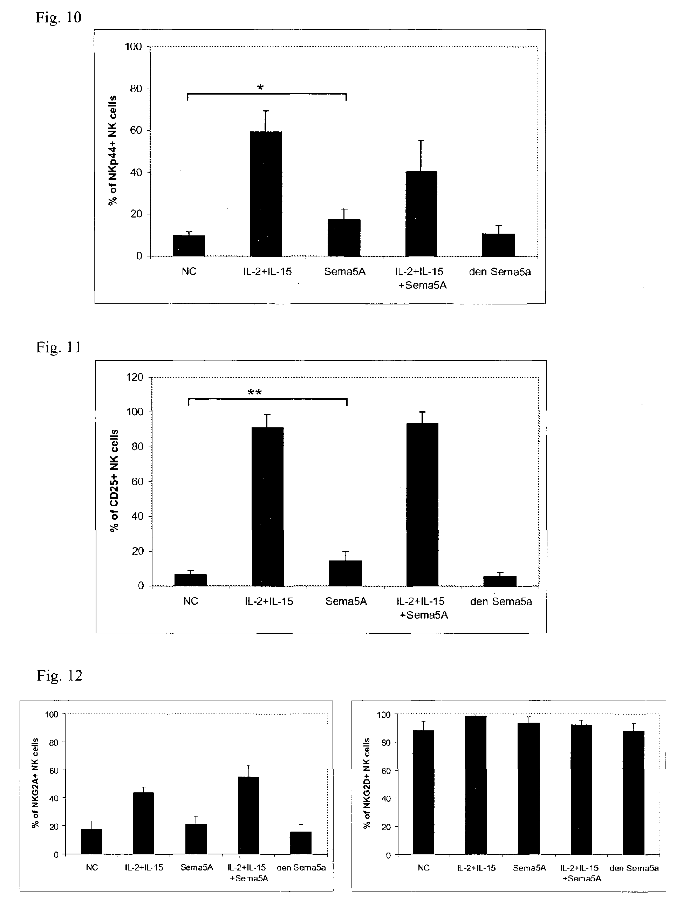

After 48 hours of cultivation, the following cell surface receptors were determined by flow cytometry specific for cell surface expression of KIR2DL1, KIR2DL2, NKp30, NKp44, NKp46, NKG2A, NKG2D, and CD25. Results are shown in Fig. 10 and demonstrate, that the exposure of the immune cells to Semaphorin 5A protein (Sema5A) leads to an increase of the natural cytotoxic receptor NKp44, i.e. NKp44+ NK cells by approximately 12%. As expression of NKp44 is restricted to activated NK cells, this result demonstrates that Semaphorin 5A protein activates NK cells. This activation of NK cells is even more pronounced for the combination of Semaphorin 5A protein with IL-2 and IL-15 (IL-2+ IL-15+Sema5A), whereas control cells (NC) without Semaphorin 5A and cells exposed to denatured Semaphorin 5A protein (den Sema5a) show lower activation.

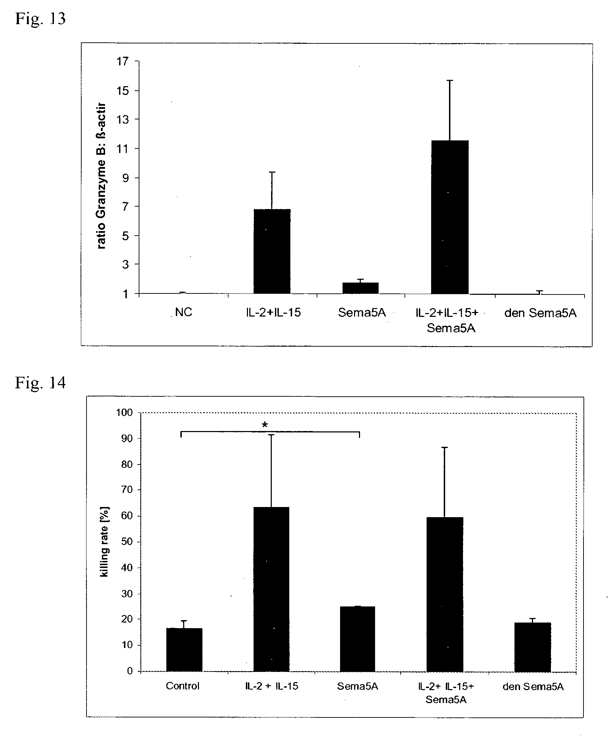

In addition, exposure of the cultivated immune cells to Semaphorin 5A stimulated an increase of the frequency of CD25⁺ NK cells by up to 20%, as shown in Fig. 11. The stimulation is even more pronounced when Semaphorin 5A is used in combination with IL-2 and IL-15. As shown in Fig. 12, the NKG2A+ (21.2%±5.7%) and NKG2D+ (93.8%±4%) NK cells increased significantly in the presence of Semaphorin 5A, when compared to NK cells that had been cultivated in the absence of Semaphorin 5A (NC), or NK cells that were incubated in the presence of Semaphorin 5A alone. No significant changes in the expression were observed for expression of NKp30, NKp46, KIR2DL1 or KIR2DL2 (data not shown).
This data show that Semaphorin 5A, e.g. in the form of recombinant Semaphorin 5A, and preferably in the presence of additional IL-2 and/or IL-15, is an activator of NK cell function. As shown in Figure 13, Semaphorin 5A stimulates NK cells to have increased levels of Granzyme B (1.72±0.31) in comparison to NK cells cultivated without Semaphorin 5A (1±0.05). mRNA levels of Granzyme B were evaluated by real-time PCR for determining its upregulation. The data shown are normalized to β-actin levels determined in aliquots.

An increased, e.g. synergistic effect for the upregulation of Granzyme B transcripts was observed for Semaphorin 5A in combination with both IL-2 and IL-15 (11.57±4.11), when compared to NK cells cultivated in IL-2 and IL-15 but without Semaphorin 5A (6.7±2.5). This clearly shows that Semaphorin 5A enhances the cytotoxic potential of NK cells, even in the absence of target cells. As a control, non-simulated NK cells were used.

In an additional experiment, the NK cells previously incubated in the presence or absence of Semaphorin 5A, as described above, were used in cytotoxicity assays with K562 cells as targets. NK cells previously cultivated in the presence of Semaphorin 5A showed an increased lysing capacity of the target cells (25%±0.3%) in comparison to NK cells cultivated in the absence of Semaphorin 5A (16.5%±3%), as is also shown in Fig. 14.

### Example 6: Stimulation of proliferation of CD8⁺ T-cells, and increase of cytotoxicity of CD8⁺ T-cells by Semaphorin 5A.

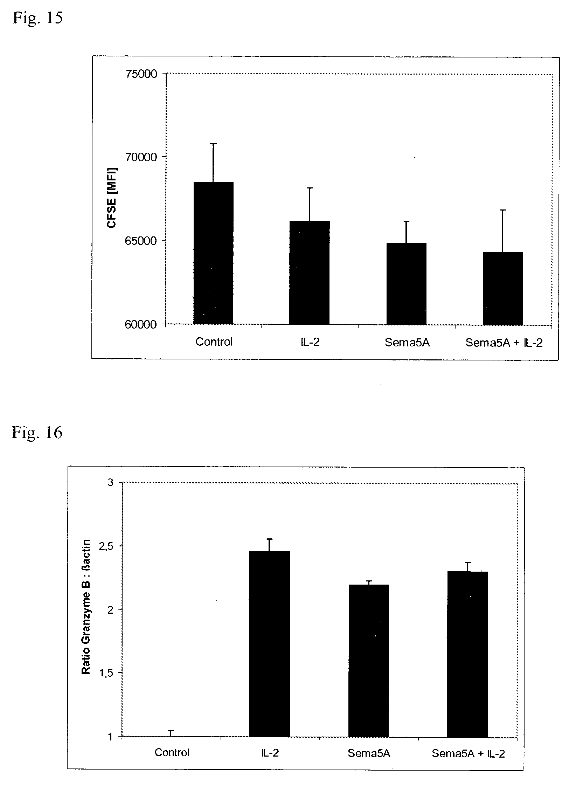

CD8⁺ T-cells were isolated from the peripheral blood of 4 healthy donors using magnetic assisted cell separation. Prior to the proliferation assays, CD8⁺ T-cells were labelled with 5,6-carboxy-succinimidyl-fluorescein-ester (CFSE), and cultured in the presence of IL-2 (50U), Semaphorin 5A (10µg) alone, or in a combination of Semaphorin 5A (10µg) and IL-2 (50U) for 5 days under standard cell cultivation conditions. As negative control in this experiment, non-stimulated cells (non-stimulated) were used, as NK cells are unable to endocytose protein and to produce cytokines in response thereto. In comparison to CD8⁺ T-cells cultivated without added stimulant (Control), cultures supplemented with IL-2 showed a 4% increase in the proliferation rate for CD8⁺ T-cells. A strong increase of the proliferation rate by about 10% was observed in the culture of CD8⁺ T-cells in the presence of Semaphorin 5A. Essentially no synergistic effect onto the proliferation rate was detected for the combination of IL-2 and Semaphorin 5A in this culture of CD8⁺ T-cells. Data are shown in Figure 15.

For an evaluation of the cytotoxic potential of CD8⁺ T-cells, total RNA was isolated from the individual cultures, and the level of Granzyme B transcripts were measured by real-time PCR. Data are shown in Fig. 16. Presence of IL-2 in the cultivation of CD8⁺ T-cells caused a 2.4±0.1 -fold increase in levels of Granzyme B. Presence of Semaphorin 5A alone, i.e. without additional IL-2, also induces an upregulation of Granzyme B-transcripts by a factor of 2.2±0.1. The combination added IL-2 and Semaphorin 5A lead to an increase of levels of Granzyme B by a factor of 2.3±0.07. These data demonstrate that it is Semaphorin 5A alone that is capable of inducing cell proliferation and an increased cytotoxicity of CD8⁺ T-cells.

Cytokines, which are known to play a crucial role in inflammatory diseases, graft rejection and GvHD were analysed by determination of the cytokine secretion profile of NK cells and monocytes obtained from healthy individuals upon addition of SEMA5A. Freshly isolated NK cells from five healthy donors were stimulated with 10µg/ml of recombinant SEMA5A in presence or absence of a combination of IL-2 (100U/mL) plus IL-15 (50ng/mL). After 2 days of culture under standard conditions, the NK cell culture supernatant was harvested and analysed for the presence of cytokines using the Luminex technology (available from Invitrogen). A significant increase in the secretion of IL-1β, IL-6 and IL-8 was detected upon stimulation of NK cells with SEMA5A in comparison to the cytokine secretion profile of non-stimulated NK cells. Furthermore, a significantly increase in the secretion of IFN-γ, IL-1β; IL-6, IL-8, TNF-α, GM-CSF was measured when NK cells were incubated in the presence of SEMA5A in combination with IL-2 and IL-15 in comparison to the cytokine levels detected in the presence of this combination of cytokines alone. Results are summarized in Table 1. No differences were observed in the secretion levels of IL-4, IL-5, IL-7, IL-10, IL-12(p70), IL-13 and MCP-1.

**Table 1: Cytokine secretion profile of NK cells incubated in the presence or absence of Sema5A**

| Added stimulant | | | | |
|---|---|---|---|---|
| Analytes (pg/ml) | Non-stimulated | IL-2 + IL-15 | Sema5A | Sema5A + IL-2 + IL-15 |
| IFN-γ | 0 | 209.07 ±141.70 | 43.56 ±52.09 | 15471 ± 4013.35 (p<0.001) |
| IL-1β | 0 | 0 | 50.00 ± 2.01 (p<0.01) | 70.10 ± 8.45 (p<0.01) |
| IL-6 | 6.66 ± 0.94 | 10.66 ±3.12 | 303.39 ±54.13 (p<0.01) | 437.51 ±0.76 (p<0.001) |
| IL-8 | 52.12 ± 44.32 | 1241.24 ± 486.12 | 4196.90 ± 1258.05 (p<0.001) | 6666.29 ± 3932 ±93 (p<0.001) |
| TNF-α | 3.64 ± 0.1 | 6.89 ± 1.32 | 6.66 ± 1.65 | 18.83 ± 8.96 (p<0.01) |
| GM-CSF | 0 | 179.46 ± 111.36 | 33.36 ± 6.94 | 523.65 ±112.9 (p<0.01) |

Further, the cytokine secretion profile of monocytes in response to SEMA5A was analysed. CD14+ cells were isolated from PBMCs of 6 healthy individuals and stimulated in presence of 10µg/mL of recombinant SEMA5A protein alone or in presence of 10µg/ml of recombinant SEMA5A in combination with 50ng/ml IFN-γ. A soluble form of a killer immunoglobulin-like receptor (KIR) protein was used as negative control. After 2 days cultivation under standard conditions, monocyte cell culture supernatants were harvested and analysed for the presence of cytokines using Luminex technology (available from Invitrogen). A significant increase in the secretion of IL-1β was observed when monocytes were stimulated in the presence of SEMA5A protein alone (3149.095 ± 2366.675 pg/mL; p<0.05) or in combination with IFN-γ (6083.278 ± 2364.507 pg/mL; p<0.05) in comparison to the levels detected in non-stimulated monocyte cultures (1227.193 ± 660.273 pg/mL) or in presence of IFN-γ (4474.031 ± 3429.2 pg/mL), respectively. Also, exposure of monocytes to a combination of SEMA5A and IFN-γ caused an augmentation in the secretion of IL-12(p70) (392.41 ± 200.153 pg/mL; p<0.05). In addition, stimulation of monocytes with SEMA5A protein alone (3028.207 ± 2321.318 pg/mL; p<0.01) or in combination with IFN-γ (11075.343 ± 806.811 pg/mL; p<0.05) resulted in a significant increase of TNF-α secretion levels in comparison to non-stimulated monocytes (1547.655 ± 743.138 pg/mL) or in presence of IFN-γ (9036.502 ± 2321.318 pg/mL). Results are summarized in Table 2, wherein NC indicates absence of SEMA5A. As a negative control (NC) in this experiment, monocytes were stimulated with another protein than SEMA5A in the presence or absence of IFN-γ in addition to non-stimulated cells (non-stimulated).

**Table 2. Cytokine secretion profile of monocytes in presence or absence of SEMA5A**

| added stimulant | | | | | | |
|---|---|---|---|---|---|---|
| Analytes (pg/mL) | Non-stimulated | IFN-γ | SEMA5A | SEMA5A + IFN-γ | NC | NC + IFN-γ |
| IL-1β | 1227.193 ± 660.273 | 4474.031 ± 3429.2 | 3149.095 ± 2366.675 (p<0.05) | 6083.278 ± 2364.507 (p<0.05) | 343.2 ± 230.1 | 3900.2 ± 859.1 |
| IL-12(p70) | 9.467 ± 3.225 | 173.029 ± 174.819 | 11.714 ± 5.386 | 392.41 ± 200.153 (p<0.05) | 3.2 ± 0.1 | 113.2 ± 20.1 |
| TNF-α | 1547.655 ± 743.138 | 9036.502 ± 2321.318 | 3028.207 ± 2321.318 (p<0.01) | 11075.343 ± 806.811 (p<0.05) | 8.823 ± 3.596 | 7930.918 ± 2645.176 |

The comparison of the NC to the specific effect of SEMA5A shows that the observed effect was caused by SEMA5A, excluding the possibility that variations in the monocyte secretion profile were a result of endocytosis of protein.

## Claims

1. Method for analysis of GvHD or HvGD in a sample of a patient, wherein the patient is a recipient of an allogeneic transplant, **characterized by** the determination of the concentration of Semaphorin 5A in a sample of the patient by detecting the mRNA encoding Semaphorin 5A or by detecting the reaction of an antibody that is specific for Semaphorin 5A protein, wherein the concentration of Semaphorin 5A is normalized to a signal determined for a constitutively expressed constituent of the sample and wherein the determined concentration of Semaphorin 5A is compared to the concentration of Semaphorin 5A determined in a healthy human, wherein an elevated concentration of Semaphorin 5A in the sample of the patient is indicative of GvHD or HvGD.

2. Method for analysis of a sample of a patient, wherein the patient is diagnosed as having or is suspected of having an autoimmune disease, **characterized by** the determination of the concentration of Semaphorin 5A in a sample of the patient by detecting the mRNA encoding Semaphorin 5A, or by detecting the reaction of an antibody that is specific for Semaphorin 5A protein wherein the concentration of Semaphorin 5A is normalized to a signal determined for a constitutively expressed constituent of the sample and the determined concentration of Semaphorin 5A is compared to the concentration of Semaphorin 5A determined in a healthy human, wherein an elevated concentration of Semaphorin 5A in the sample of the patient indicates an acute phase of the autoimmune disease.

3. Method according to one of the preceding claims, **characterized in that** cancer patients are excluded.

4. Use of a kit of components in a method according to one of the preceding claims, the kit comprising a pair of oligonucleotides specifically hybridising to a nucleotide sequence containing at least a fraction of the nucleotide sequence encoding Semaphorin 5A.

5. Use according to claim 4, **characterized in that** the kit further comprises a pair of oligonucleotides specifically hybridising to a nucleotide sequence encoding at least a fraction of a constitutively expressed mammalian protein.

6. Use of a kit of components for use in a method according to one of claims 1 to 2, the kit comprising an antibody which is specific for Semaphorin 5A protein.

7. Use according to claim 6, **characterized in that** the kit further comprises an antibody which is specific for a constitutively expressed mammalian protein.

8. Semaphorin 5A protein for use as a medicament for increasing the proliferative and cytotoxic activity of NK cells and for increasing the proliferation and cytotoxic activity of CD8+ T- cells in a patient, **characterized in that** the patient is free from cancer.

9. Semaphorin 5A for use according to claim 8, **characterized in that** Semaphorin 5A has an amino acid sequence according to SEQ ID NO 11.

10. Semaphorin 5A for use according to one of claims 8 or 9, **characterized in that** Semaphorin 5A is in combination with IL-2 and/or IL-15.

## Patentansprüche

1. Verfahren zur Analyse der GvHD oder HvGD in einer Probe eines Patienten, bei dem der Patient ein Empfänger eines allogenen Transplantats ist, **gekennzeichnet durch** die Bestimmung der Konzentration von Semaphorin 5A in einer Probe des Patienten durch Detektieren der mRNA, die Semaphorin 5A kodiert oder **durch** Detektieren der Reaktion eines Antikörpers, der für das Semaphorin 5A-Protein spezifisch ist, wobei die Konzentration von Semaphorin 5A zu einem Signal normalisiert wird, das für einen konstitutiv exprimierten Bestandteil der Probe bestimmt wird und wobei die festgestellte Konzentration von Semaphorin 5A mit der Konzentration von Semaphorin 5A verglichen wird, die in einem gesunden Menschen bestimmt ist, wobei eine erhöhte Konzentration von Semaphorin 5A in der Probe des Patienten GvHD oder HvGD anzeigt.

2. Verfahren zur Analyse einer Probe eines Patienten, bei der der Patient dahingehend diagnostiziert ist, dass er eine Autoimmunkrankheit hat oder dieser verdächtig ist, **gekennzeichnet durch** die Bestimmung der Konzentration von Semaphorin 5A in einer Probe des Patienten **durch** Detektieren der mRNA, die Semaphorin 5A kodiert, oder durch Detektieren der Reaktion eines Antikörpers, der spezifisch für das Semaphorin 5A-Protein ist, wobei die Konzentration von Semaphorin 5A zu einem Signal normalisiert wird, das für einen konstitutiv exprimierten Bestandteil der Probe bestimmt wurde und die festgestellte Konzentration von Semaphorin 5A mit der Konzentration von Semaphorin 5A verglichen wird, die in einem gesunden Menschen bestimmt wurde, wobei eine erhöhte Konzentration von Semaphorin 5A in der Probe des Patienten eine akute Phase der Autoimmunkrankheit anzeigt.

3. Verfahren nach einem der voranstehenden Ansprüche, **dadurch gekennzeichnet, dass** Krebspatienten ausgeschlossen sind.

4. Verwendung eines Kits von Bestandteilen in einem Verfahren nach einem der voranstehenden Ansprüche, wobei das Kit ein Paar von Oligonukleotiden umfasst, die spezifisch mit einer Nukleotidsequenz hybridisieren, die wenigstens einen Anteil der Semaphorin 5A kodierenden Nukleotidsequenz enthält.

5. Verwendung nach Anspruch 4, **dadurch gekennzeichnet, dass** das Kit überdies ein paar Oligonukleotide umfasst, die spezifisch mit einer Nukleotidsequenz hybridisieren, die wenigstens einen Anteil eines konstitutiv exprimierten Säugerproteins kodiert.

6. Verwendung eines Kits von Bestandteilen zur Verwendung in einem Verfahren nach einem der Ansprüche 1 bis 2, wobei das Kit einen Antikörper umfasst, der für Semaphorin 5A Protein spezifisch ist.

7. Verwendung nach Anspruch 6, **dadurch gekennzeichnet, dass** das Kit überdies einen Antikörper umfasst, der für ein konstitutiv exprimiertes Säugerprotein spezifisch ist.

8. Semaphorin 5A-Protein zur Verwendung als ein Medikament zur Steigerung der poliferativen und zytotoxischen Aktivität von NK-Zellen und zur Erhöhung der Proliferation und zytotoxischen Aktivität von CD8+ T-Zellen in einem Patienten, **dadurch gekennzeichnet, dass** der Patient frei von Krebs ist.

9. Semaphorin 5A zur Verwendung nach Anspruch 8, **dadurch gekennzeichnet, dass** das Semaphorin 5A eine Aminosäuresequenz gemäß SEQ ID NO 11 aufweist.

10. Semaphorin 5A zur Verwendung nach einem der Ansprüche 8 oder 9, **dadurch gekennzeichnet, dass** Semaphorin 5A in Kombination mit IL-2 und/oder IL-15 vorliegt.

## Revendications

1. Procédé d'analyse de GvHD ou HvGD dans un échantillon d'un patient, dans lequel le patient est un receveur d'une allogreffe, **caractérisé par** la détermination de la concentration de sémaphorine 5A dans un échantillon du patient par détection d'ARNm codant pour la sémaphorine 5A ou par détection de la réaction d'un anticorps qui est spécifique de la protéine de sémaphorine 5A, dans laquelle la concentration de sémaphorine 5A est normalisée par rapport à un signal déterminé pour un constituant exprimé de manière constitutive de l'échantillon et dans laquelle la concentration déterminée de sémaphorine 5A est comparée à la concentration de sémaphorine 5A déterminée chez un être humain en bonne santé, dans lequel une concentration élevée de sémaphorine 5A dans l'échantillon du patient est indicative de GvHD ou HvGD.

2. Procédé d'analyse d'un échantillon d'un patient, dans lequel le patient est diagnostiqué comme ayant ou étant soupçonné d'avoir une maladie auto-immunitaire, **caractérisé par** la détermination de la concentration de sémaphorine 5A dans un échantillon du patient par détection de l'ARNm codant la sémaphorine 5A, ou par détection de la réaction d'un anticorps qui est spécifique de la protéine sémaphorine 5A dans laquelle la concentration de sémaphorine 5A est normalisée par rapport à un signal déterminé pour un constituant exprimé de manière constitutive de l'échantillon et la concentration déterminée de sémaphorine 5A est comparée à la concentration de sémaphorine 5A déterminée chez un être humain en bonne santé, dans lequel une concentration élevée de sémaphorine 5A dans l'échantillon du patient indique une phase aiguë de la maladie auto-immunitaire.

3. Procédé selon l'une quelconque des revendications précédentes, **caractérisé en ce que** les patients atteints de cancer sont exclus.

4. Utilisation d'un kit de composants dans un procédé selon l'une des revendications précédentes, le kit comprenant une paire d'oligonucléotides s'hybridant de manière spécifique pour donner une séquence nucléotidique contenant au moins une fraction de la séquence nucléotidique codant la sémaphorine 5A.

5. Utilisation selon la revendication 4, **caractérisé en ce que** le kit comprend en outre une paire d'oligonucléotides s'hybridant de manière spécifique pour donner une séquence nucléotidique contenant au moins une fraction d'une protéine de mammifère exprimée de manière constitutive.

6. Utilisation d'un kit de composants pour une utilisation dans un procédé selon l'une des revendications 1 à 2, le kit comprenant un anticorps qui est spécifique à la protéine sémaphorine 5A.

7. Utilisation selon la revendication 6, **caractérisée en ce que** le kit comprend en outre un anticorps qui est spécifique à une protéine de mammifère exprimée de manière constitutive.

8. Protéine sémaphorine 5A pour utilisation comme médicament permettant d'augmenter l'activité proliférative et cytotoxique des cellules NK et permettant d'augmenter l'activité proliférative et cytotoxique des cellules CD8+ T chez un patient, **caractérisée en ce que** le patient n'est pas atteint d'un cancer.

9. Sémaphorine 5A pour utilisation selon la revendication 8, **caractérisée en ce que** la sémaphorine 5A présente une séquence d'acides aminés selon SED ID n°11.

10. Sémaphorine 5A pour utilisation selon l'une des revendications 8 ou 9, **caractérisé en ce que** la sémaphorine 5A est en combinaison avec IL-2 et/ou IL-15.
